# EUROPEAN PATENT APPLICATION

(11) **EP 4 335 864 A1**
(43) Date of publication of application: **13.03.2024**
(21) Application number: 22382828.6
(22) Date of filing: 06.09.2022
(51) Int. Cl.: C07K 14/005, A61P 37/00, C07K 14/47, A61K 38/00

(54) **INTERFERON INHIBITOR AND ITS USE IN THE TREATMENT OF AUTOINFLAMMATORY DISEASES**

(71) Applicant: Consejo Superior de Investigaciones Científicas (CSIC), 28006 Madrid (ES)
(72) Inventor: Alcamí, Pertejo, Antonio Javier, Madrid (ES); Hernáez de la Plaza, Bruno, Madrid (ES); Álvarez de Miranda, Rodriguez, Francisco Javier, Madrid (ES)
(74) Representative: Pons

(57) **Abstract**

The present invention relates to an isolated amino acid sequence comprising an amino acid sequence having a sequence identity of at least 70% with the sequence SEQ ID NO: 1, or the nucleotide sequence encoding thereof, for use as a medicament, preferably, for use in the treatment of an autoinflammatory disorder in a subject, with the proviso that the isolated amino acid sequence is not the sequence SEQ ID NO: 2, or SEQ ID NO: 3. The invention also relates to a fusion protein and its use in the treatment of autoinflammatory disorder in a subject.

## Description

The invention relates to an isolated amino acid sequence comprising an amino acid sequence having a sequence identity of at least 70% with the sequence SEQ ID NO: 1, or the nucleotide sequence encoding thereof, for use in the treatment of an autoinflammatory disorder in a subject. Thus, the present invention relates to the clinical field, specifically, to the field of the treatment of autoinflammatory diseases.

### BACKGROUND ART

Innate immunity is the first line of defense in vertebrates when facing a viral infection. Type I interferon (IFN-I) is a family of early induced proinflammatory cytokines that are mainly secreted from infected cells after sensing viral products. IFN-I is recognized in healthy cells through the type I IFN receptor (IFNAR) and transmits its signal through the JAK/STAT signaling pathway to stimulate the transcription of interferon stimulated genes that collaborate in the antiviral response.

Extended coexistence of viral offence and host defense through evolution has allowed viruses to develop evasion mechanisms to evade the immune response. Orthopoxviruses provide good examples of IFN-I evasion, with most members of this family exhibiting multiple mechanisms to reduce its antiviral action. Among these strategies, the secretion of viral IFN-I binding proteins (vIFNα/βBPs) from infected cells prevents recognition of several IFN-I forms by its cellular receptor IFNAR, thus acting as a decoy receptor. This has proven to be an efficient and direct way to counteract IFN-I. vIFNα/βBPs are highly conserved among orthopoxviruses, with variola virus, monkeypox virus or ectromelia virus encoding similar receptors, being B18 protein from vaccinia virus (VACV) the most studied. These proteins are composed of 3 immunoglobulin-like domains (D1, D2 and D3) and can bind to glycosaminoglycans (GAGs) at the surface of infected and uninfected cells.

Despite its beneficial role in antiviral defense, dysregulation of the IFN-I pathway is the main cause of the pathology in a group of autoinflammatory disorders known as interferonopathies, such as systemic lupus erythematosus or Aicardi-Goutières syndrome. This makes IFN-I an interesting and promising therapeutic target. Current treatments for these disorders are mainly focused on blocking IFN-I through specific antibodies against IFNAR or IFN-I itself, but with incomplete success (Amezcua-Guerra et al., 2015. Rheumatology (Oxford, England), 54(2), 203-205). In a similar way, tumor necrosis factor alpha (TNFα) is a proinflammatory cytokine that, when dysregulated, leads to autoimmune disorders. Clinical treatment for these diseases includes four different anti TNFα antibodies and Etanercept, a soluble version of the cellular TNFα receptor (Mitoma et al., 2018. Cytokine, 101, 56-63; Tak & Kalden, 2011. Arthritis Research & Therapy, 13 Suppl 1, S5). To date, no soluble IFN-I receptors have been developed to be used in the clinic. Consequently, there is a necessity in the state of the art to provide new compounds for the treatment for these disorders alternatively to those focused on blocking IFN-I through specific antibodies against IFNAR or IFN-I itself.

### DESCRIPTION OF THE INVENTION

The inventors have found that the D3 immunoglobulin-like domain (D3 domain) from the B18 protein (a viral IFN-I binding protein) coming from vaccinia virus retains, unexpectedly, the complete B18 protein ability to bind and block IFN-I. Advantageously, this D3 domain (i) shows a lower immunogenicity than the viral proteins and, at the same time, (ii) it is capable of binding multiple forms of IFN-I with a higher affinity than monoclonal antibodies thus acting as an interferon inhibitor, (iii) it is soluble, and (iv) could be humanized more easily. These properties (i) to (iv) turn D3 domain into a potent anti-inflammatory medicament to be used in clinic, particularly, in the treatment of autoinflammatory disorders known as interferonopathies, such as systemic lupus erythrematosus or Aicardi-Goutières syndrome.

To that purpose, the inventors first determined whether D3 domain from B18 protein retains the ability to bind and block IFN-I, which would make the minimal IFN-I decoy receptor. This minimal decoy receptor was then fused to a GAG binding domain combining both vIFNα/βBP functions in a smaller protein, constituting a new line of research in the development of IFN-I therapies.

### Amino acid sequence of the invention for use in the treatment of autoinflammatorv disorders.

In one aspect, the present invention relates to an isolated amino acid sequence, hereinafter "amino acid sequence of the invention", comprising, or consisting of, an amino acid sequence, or a fragment thereof, having a sequence identity of at least 70, 75, 80, 85, 90, 91, 92, 93, 94, 95, 96, 97, 98, or 99 % with the sequence SEQ ID NO: 1 (D3 domain), or the nucleotide sequence encoding thereof, for use as a medicament, preferably, for use in the treatment of an autoinflammatory disorder in a subject (hereinafter, "use of the invention"), with the proviso that the isolated amino acid sequence is not the sequence SEQ ID NO: 2, or SEQ ID NO: 3.

The amino acid sequence SEQ ID NO: 1 relates to the isolated sequence of the D3 immunoglobulin-like domain from the B18 protein coming from vaccinia virus, showing the amino acid sequence:

FKLILDPKINVTIGEPANITCTAVSTSLLIDDVLIEWENPSGWLIGFDFDVYSVLTS
RGGITEATLYFENVTEEYIGNTYKCRGHNYYFEKTLTTTVVLE

As the skilled person in the art understands, the present invention also encompasses all those variants of said sequence SEQ ID NO: 1 which, even having different amino acid sequence, show the same properties than the sequence SEQ ID NO: 1 (properties (i) to (iv) cited above) and as a consequence, show the same functionality, i.e.: they are potent anti-inflammatory medicaments which can be used in clinic, particularly, in the treatment of autoinflammatory disorders. Illustrative examples of these variants are, without limited to, the D3 immunoglobulin-like domain from the IFN-I binding proteins coming from poxviruses such as Ectromelia virus Naval (GenBank, AIF30251.1), Monkeypox virus UK-P2 (GenBank, QNP13770.1), and Variola virus strain V77-1605 (GenBank, ABF27576.1). Non limitative examples of D3 immunoglobulin-like domains from said poxviruses are the sequences SEQ ID NO: 31, SEQ ID NO: 32 and SEQ ID NO: 33 below.
SEQ ID NO: 31 (Ectromelia virus Naval) 
FKLILDPKINVTIGEPANITCTAVSTSLLVDDVLIDWENPSGWIIGLDFGVYSILTS
SGGITEATLYFENVTEEYIGNTYTCRGHNYYFDKTLTTTVVLE
SEQ ID NO: 32 (Monkeypox virus UK-P2) 
FKLILDPKINVTIGEPANITCSAVSTSLFVDDVLIEWENPSGWIIGLDFGVYSILTS
RGGITEATLYFENVTEEYIGNTYTCRGHNYYFDKTLTTTVVLE
SEQ ID NO: 33 (Variola virus strain V77-1605)

FKLILDSKINVIIGEPANITCTAVSTSLLFDDVLIEWENPSGWLIGFNFDVYSVLTS
RGGITEASLYFKNVTEEYIGNTYKCRGHNYYFEKTLTTTVVLE

All these proteins are functionally equivalent variants of SEQ ID NO: 1. An example of an assay to test if a given amino acid sequence is a functionally equivalent variant of the sequence SEQ ID NO: 1 is disclosed in the Example illustrative of the present invention.

In the context of the present invention, the term "sequence identity" or "identity" is understood to mean the degree of similarity between two nucleotide or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality. Thus, as cited above, variants of the amino acid sequence SEQ ID NO: 1 are encompassed within the context of the present invention.

The present invention also encompasses fragments of the amino acid sequence of the invention, being said fragments considered variants of the amino acid sequence SEQ ID NO: 1. In the context of the present invention, the term "fragment" refers to an amino acid sequence having one or more (several) amino acids deleted from the amino and/or carboxyl terminus of the sequence SEQ ID NO: 1; wherein the fragment shows the same properties than the sequence SEQ ID NO: 1 (properties (i) to (iv) cited above) and as a consequence, show the same functionality, i.e. they are potent anti-inflammatory medicaments which can be used in clinic, particularly, in the treatment of autoinflammatory disorders.

In a particular embodiment, the present invention relates to an isolated amino acid sequence comprising an amino acid sequence, or a fragment thereof, having a sequence identity of 100% with the sequence SEQ ID NO: 1 (D3 domain), or the nucleotide sequence encoding thereof, for use as a medicament, preferably, for use in the treatment of an autoinflammatory disorder in a subject, with the proviso that the isolated amino acid sequence is not the sequence SEQ ID NO: 2, or SEQ ID NO: 3.

In another particular embodiment, the present invention relates to an isolated amino acid sequence consisting of an amino acid sequence, or a fragment thereof, having a sequence identity of at least 70, 75, 80, 85, 90, 91, 92, 93, 94, 95, 96, 97, 98, 99 o 100% with the sequence SEQ ID NO: 1 (D3 domain), or the nucleotide sequence encoding thereof, for use as a medicament, preferably, for use in the treatment of an autoinflammatory disorder in a subject.

In another particular embodiment, the present invention relates to an isolated amino acid sequence consisting of an amino acid sequence, or a fragment thereof, having a sequence identity of 100% with the sequence SEQ ID NO: 1 (D3 domain), or the nucleotide sequence encoding thereof, for use as a medicament, preferably, for use in the treatment of an autoinflammatory disorder in a subject.

In addition, the inventors realized that if a cell binding surface is linked to the D3 domain, the resulting amino acid sequence not only retains the B18 full protein ability to bind and block IFN-I, but it is also retained around the site of infection enhancing the immunomodulatory activity of D3.

Consequently, in a particular embodiment of the amino acid sequence of the invention, alone or in combination with each of the previous particular embodiments, the amino acid sequence further comprises a cell surface binding domain.

As use herein, the term "cell surface binding domain" refers to the region within the first immunoglobulin-like domain of the IFN-I binding protein that functions to bind the IFN-I binding protein to cell surfaces.

Any cell binding domain can be used in the context of the present invention. Examples of said cell binding domains include, without limiting to, those present in apoE, fibronectin, laminin, aggrecan and antithrombin. Nevertheless, in a particular embodiment, alone or in combination with each one of the previous particular embodiments, the cell surface binding domain is a Glycosaminoglycan (GAG)-binding domain.

As used herein, the term "GAG-binding domain" refers to a region within a protein that functions to bind a protein to cell surface glycosaminoglycans

In a more particular embodiment of the amino acid sequence of the present invention, the cell surface binding domain is
(a) an amino acid sequence having a sequence identity of at least 70, 80, 90, 91, 92, 93, 94, 95, 96, 97, 98, 99 o 100% with the sequence SEQ ID NO: 4 (GAG-BD), or
(b) an amino acid sequence having a sequence identity of at least 70, 80, 90, 91, 92, 93, 94, 95, 96, 97, 98, 99 o 100% with the sequence SEQ ID NO: 5 (full D1 from B18), or
(c) an amino acid sequence having a sequence identity of at least 70, 80, 90, 91, 92, 93, 94, 95, 96, 97, 98, 99 o 100% with the sequence SEQ ID NO: 6 (MC MC54L GAG-BD).

The term "sequence identity" has been defined in previous paragraphs.

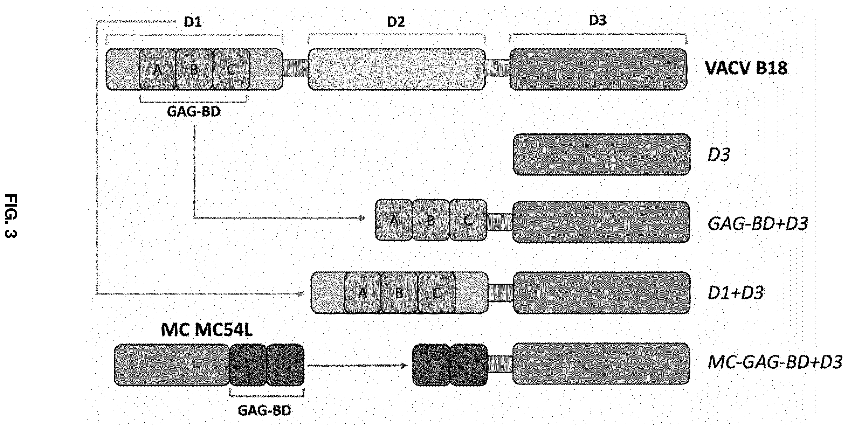

In the present invention, the sequence SEQ ID NO: 4 corresponds to the GAG-cell binding domain of the B18 protein coming from vaccinia virus (see Figure 3), having the following amino acid sequence:

KWERLEKNRRRQVSNKRVKHGDLWIANYTSKFSNRRYLCTVTTKNGDCVQGIVRSHI

RK

The sequence SEQ ID NO: 4 is encoded by the nucleotide sequence SEQ ID NO: 7:

aaatgggaaaggctagaaaaaaatagacggcgacaggtttctaataaacgtgttaaa
catggtgatttatggatagccaactatacatctaaattcagtaaccgtaggtatttg
tgcaccgtaactacaaagaatggtgactgtgttcagggtatagttagatctcatatt
agaaaa

Alternatively, the cell binding domain may have the sequence SEQ ID NO: 5 corresponding to the whole cell binding domain of B18 protein coming from vaccinia virus, also called D1 domain (see Figure 3). The SEQ ID NO: 5 has the following amino acid sequence:

IDIENEITEFFNKMRDTLPAKDSKWLNPACMFGGTMNDIAALGEPFSAKCPPIEDSL
LSHRYKDYVVKWERLEKNRRRQVSNKRVKHGDLWIANYTSKFSNRRYLCTVTTKNGD
CVQGIVRSHIRKVD

The sequence SEQ ID NO: 5 is encoded by the nucleotide sequence SEQ ID NO: 8:

atagacatcgaaaatgaaatcacagaattcttcaataaaatgagagatactctacca
gctaaagactctaaatggttgaatccagcatgtatgttcggaggcacaatgaatgat
atagccgctctaggagagccattcagcgcaaagtgtcctcctattgaagacagtctt
ttatcgcacagatataaagactatgtggttaaatgggaaaggctagaaaaaaataga
cggcgacaggtttctaataaacgtgttaaacatggtgatttatggatagccaactat
acatctaaattcagtaaccgtaggtatttgtgcaccgtaactacaaagaatggtgac
tgtgttcagggtatagttagatctcatattagaaaagtcgac

Finally, the cell binding domain may have the sequence SEQ ID NO: 6 corresponding to the cell binding domain from *Molluscum contagiosum* virus MC54L protein, and having the amino acid sequence:

PPGAPTSPGAPTLPGAPKVVGTPPPKGNKKNKKKGKGKKRRGNKTKHKNDKKRGGIP

KNRHVRVD

The sequence SEQ ID NO: 6 is encoded by the nucleotide sequence SEQ ID NO: 9:

ccgccgggcgcgccgacgtcaccggcgcgccaacgctgccgggcgcgccaaaggtt
gtaggaactccacctccaaaaggaacaagaagaacaagaagaaaggcaaaggaaag
aaacgccgcggaaacaagacaaaacacaagaatgataagaaaagggggcataccc
aagaatcgacatgtcaggtcgac

As the skilled person understands, the present invention also encompasses variants of these cell binding domains which, even having different amino acid sequence, show the same capacity that the corresponding sequences SEQ ID NO: 4, SEQ ID NO: 5 and SEQ ID NO: 6, i.e. they are functionally equivalent variants capable of binding vIFNα/βBPs and enhancing the immunomodulatory activity of D3. An example of an assay to test if a given cell binding domain is a functionally equivalent variant of the sequences SEQ ID NO: 4, SEQ ID NO: 5 or SEQ ID NO: 6 is disclosed in the illustrative Example of the present invention.

The D3 domain domain from the B18 protein coming from vaccinia virus, or any of its functionally equivalent variants as defined above, may be covalently bound to the cell binding domain. The terms "bound to", "couple to" and "linked to" are considered equivalents and can be used interchangeably throughout the present description. The expression "covalently bound to each other" means that the two amino acid sequences are bound by a bond that involves the sharing of electron pairs between atoms, such as a peptide bond. On the contrary, the expression "non-covalently bound to each other" means that the two amino acid sequences are bound by a bond in that it does not involve the sharing of electrons, but rather involves more dispersed variations of electromagnetic interactions between molecules or within a molecule. Likewise, the D3 domain domain from the B18 protein coming from vaccinia virus, or any of its functionally equivalent variants as defined above, may be bound through a linker. Thus, in another particular embodiment of the amino acid sequence of the invention, alone or in combination with each of the previous particular embodiments, the isolated amino acid sequence comprises a linker between the sequence SEQ ID NO: 1 and the cell surface binding domain.

As used herein, the term "linker" or "peptide linker" refers to a peptide sequence linked to either the N-terminus or the C-terminus of a polypeptide sequence. Any "linker" group is optional. When present, its chemical structure is not critical, since it serves primarily as a spacer. The linker is preferably made up of amino acids linked together by peptide bonds. Thus, in preferred embodiments, the linker is made up of from 1 to 20 amino acids linked by peptide bonds, wherein the amino acids are selected from the 20 naturally occurring amino acids. Some of these amino acids may be glycosylated, as is well understood by those in the art. In a more preferred embodiment, the 1 to 20 amino acids are selected from glycine, alanine, proline, asparagine, glutamine, and lysine. Even more preferably, a linker is made up of a majority of amino acids that are sterically unhindered, such as glycine and alanine. In order to ensure proper folding of the protein, these linkers should not contain truncated α-helices or β-sheets. Many linkers are known from the state of the art and any of them can be used in the context of the present invention. Examples of linkers include, without limited to, GGGGGGGG (SEQ ID NO: 34), AEAAAKEAAAKEAAAKEAAAKALEAEAAAKEAAAKEAAAKEAAAKA (SEQ ID NO: 35) and APAPAPAPAPAPAPAPAPAP (SEQ ID NO: 36).

In a particular embodiment, the linker used in the present invention comprise the sequence SEQ ID NO: 10.
SEQ ID NO: 10: GGGGSGGGGSGGGGS

As has been explained in previous paragraphs, the isolated amino acid sequence of the invention may comprise a lot of different amino acid sequences, but all of them having the same property, i.e.: there are multiple functional equivalent variants of the amino acid sequence of the invention as explained in previous paragraphs, and all of them fall within the scope of the invention disclosed herein. Nevertheless, in a particular embodiment, the isolated amino acid sequence of the invention comprises, or consists of, the sequence SEQ ID NO: 11 (GAG-BD+D3), SEQ ID NO: 13 (D1+D3) or SEQ ID NO: 15 (MC-GAG-BD+D3).
SEQ ID NO: 11

KWERLEKNRRRQVSNKRVKHGDLWIANYTSKFSNRRYLCTVTTKNGDCVQGIVRSHI
RKGGGGSGGGGSGGGGSVDHRFKLILDPKINVTIGEPANITCTAVSTSLLIDDVLIE
WENPSGWLIGFDFDVYSVLTSRGGITEATLYFENVTEEYIGNTYKCRGHNYYFEKTL
TTTVVLE

The nucleotide sequence encoding the SEQ ID NO: 11 comprise, or consists of, the sequence SEQ ID NO: 12: 
aaatgggaaaggctagaaaaaaatagacggcgacaggtttctaataaacgtgttaaa
catggtgatttatggatagccaactatacatctaaattcagtaaccgtaggtatttg
tgcaccgtaactacaaagaatggtgactgtgttcagggtatagttagatctcatatt
agaaaaggtggcggaggttctggtggcggcggtagcggtggcggtggttccgtcgac
cacaggtttaaactaatactagatccaaaaatcaacgtaacgataggagaacctgcc
aatataacatgcactgctgtgtcaacgtcattattgattgacgatgtactgattgaa
tgggaaaatccatccggatggcttataggattcgattttgatgtatactctgtttta
actagtagaggcggtattaccgaggcgaccttgtactttgaaaatgttactgaagaa
tatataggtaatacatataaatgtcgtggacacaactattattttgaaaaaaccctt
acaactacagtagtattggag
 SEQ ID NO: 13

IDIENEITEFFNKMRDTLPAKDSKWLNPACMFGGTMNDIAALGEPFSAKCPPIEDSL
LSHRYKDYVVKWERLEKNRRRQVSNKRVKHGDLWIANYTSKFSNRRYLCTVTTKNGD
CVQGIVRSHIRKVDGGGGSGGGGSGGGGSHRFKLILDPKINVTIGEPANITCTAVST
SLLIDDVLIEWENPSGWLIGFDFDVYSVLTSRGGITEATLYFENVTEEYIGNTYKCR
GHNYYFEKTLTTTVVLE

The nucleotide sequence encoding the SEQ ID NO: 13 comprise, or consists of, the sequence SEQ ID NO: 14: 
atagacatcgaaaatgaaatcacagaattcttcaataaaatgagagatactctacca
gctaaagactctaaatggttgaatccagcatgtatgttcggaggcacaatgaatgat
atagccgctctaggagagccattcagcgcaaagtgtcctcctattgaagacagtctt
ttatcgcacagatataaagactatgtggttaaatgggaaaggctagaaaaaaataga
cggcgacaggtttctaataaacgtgttaaacatggtgatttatggatagccaactat
 
acatctaaattcagtaaccgtaggtatttgtgcaccgtaactacaaagaatggtgac
tgtgttcagggtatagttagatctcatattagaaaagtcgacggtggcggaggttct
ggtggcggcggtagcggtggcggtggttcccacaggtttaaactaatactagatcca
aaaatcaacgtaacgataggagaacctgccaatataacatgcactgctgtgtcaacg
tcattattgattgacgatgtactgattgaatgggaaaatccatccggatggcttata
ggattcgattttgatgtatactctgttttaactagtagaggcggtattaccgaggcg
accttgtactttgaaaatgttactgaagaatatataggtaatacatataaatgtcgt
ggacacaactattattttgaaaaaacccttacaactacagtagtattggag
 SEQ ID NO: 15

PPGAPTSPGAPTLPGAPKVVGTPPPKGNKKNKKKGKGKKRRGNKTKHKNDKKRGGIP
KNRHVRVDGGGGSGGGGSGGGGSHRFKLILDPKINVTIGEPANITCTAVSTSLLIDD
VLIEWENPSGWLIGFDFDVYSVLTSRGGITEATLYFENVTEEYIGNTYKCRGHNYYF
EKTLTTTVVLE

The nucleotide sequence encoding the SEQ ID NO: 15 comprise, or consists of, the sequence SEQ ID NO: 16:

ccgccgggcgcgccgacgtcaccgggcgcgccaacgctgccgggcgcgccaaaggttgtagg
aactccacctccaaaagggaacaagaagaacaagaagaaaggcaaaggaaagaaacgccgcg
gaaacaagacaaaacacaagaatgataagaaaagggggggcatacccaagaatcgacatgtc
agggtcgacggtggcggaggttctggtggcggtagcggtggcggtggttcccacaggtt
taaactaatactagatccaaaaatcaacgtaacgataggagaacctgccaatataacatgca
ctgctgtgtcaacgtcattattgattgacgatgtactgattgaatgggaaaatccatccgga
tggcttataggattcgattttgatgtatactctgttttaactagtagaggcggtattaccga
ggcgaccttgtactttgaaaatgttactgaagaatatataggtaatacatataaatgtcgtg
gacacaactattattttgaaaaaacccttacaactacagtagtattggag

The peptide of the invention may be of any length. Nevertheless, in a particular embodiment, alone or in combination with all or each one of the previous particular embodiments, the length of the peptide is from 100 to 400 amino acids, or from 100 to 350 amino acids, or from 100 to 300 amino acids, or from 100 to 250 amino acids or from 100 to 200 amino acids, or from 100 to 150 amino acids.

In the present invention, both the amino acid sequence of the invention and the nucleotide sequence encoding thereof can be used as a medicament or in the treatment an autoinflammatory disorder. Thus, in a particular embodiment, the nucleotide sequence encoding the amino acid sequence of the invention comprise, or consists of, the sequence SEQ ID NO: 37, SEQ ID NO: 12, SEQ ID NO: 14 or SEQ ID NO: 16 (hereinafter "nucleotide sequence of the invention"). Nucleotide sequence SEQ ID NO: 37 is the nucleotide sequence encoding the amino acid sequence SEQ ID NO: 1, and shows the sequence

tttaaactaatactagatccaaaaatcaacgtaacgataggagaacctgccaatataacatg
cactgctgtgtcaacgtcattattgattgacgatgtactgattgaatgggaaaatccatccg
gatggcttataggattcgattttgatgtatactctgttttaactagtagaggcggtattacc
gaggcgaccttgtactttgaaaatgttactgaagaatatataggtaatacatataaatgtcg
tggacacaactattattttgaaaaaacccttacaactacagtagtattggag

Nucleotide sequences SEQ ID NO: 12, SEQ ID NO: 14 and SEQ ID NO: 16 have been disclosed above.

In a particular embodiment, the nucleotide sequence encoding the amino acid sequences of the invention for use as a medicament, or for use in the treatment of an autoinflammatory disease, is not the nucleotide sequence SEQ ID NO: 18 (Nucleotide sequence encoding B18 full protein).

The nucleotide sequence of the invention may be inserted into a vector, and this vector into a cell for use as a medicament or for use in the treatment of an autoinflammatory disease. Likewise, the amino acid sequence of the invention, or the nucleotide sequence encoding thereof (as well as the vector and the cell comprising it) may be comprised into a composition, particularly, a pharmaceutical composition, for use in in the treatment of an autoinflammatory disease. The different embodiments and definitions of the vectors, cell and compositions which can be used in the context of the present invention, will be disclosed in detail below with the next inventive aspect.

As explained in the beginning of the present description, in one aspect the present invention relates to the amino acid sequence of the invention as defined in previous paragraphs, for use as a medicament, preferably, for use in the treatment of an autoinflammatory disorder in a subject.

The term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. It is to be understood that a subject/patient may experience a broad range of responses to a treatment (such as the exemplary responses as described herein above).

As used herein, the term "autoinflammatory disorder" or "autoinflammatory disease" refers to the medical condition or issue that are caused by, or exacerbated by, a malfunction of the immune system (inflammation). Examples of autoinflammatory disorders include, without limited to, cardiovascular disease, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, hypercoagulation, hemophilia, ulcers, wounds, lesions, cuts, abrasions, oxidative damage, age- related tissue degeneration, surgically related lesions, burns, muscle weakness, muscle atrophy, connective tissue disorders, idiopathic thrombocytopenic purpura, heart failure, secondary pathologies caused by heart failure and hypertension, hypotension, angina pectoris, myocardial infarction, tuberous sclerosis, scleroderma, transplantation, inflammation, chronic and acute conditions, including chronic prostatitis, granulomatous prostatitis and malacoplakia, inflammation associated with infection (e.g., septic shock, sepsis, or systemic inflammatory response syndrome (SIRS)), ischemia-reperfusion injury, endotoxin lethality, arthritis, complement- mediated hyperacute rejection, nephritis, cytokine or chemokine induced lung injury, inflammatory bowel disease, Crohn's disease, or resulting from over production of cytokines (e.g., TNF or IL-1.) Hyperproliferative Disorders, neoplasms, autoimmune disease, cystic fibrosis, allergic reactions and conditions, such as asthma (particularly allergic asthma) or other respiratory problems, hemolytic anemia, antiphospholipid syndrome, rheumatoid arthritis, dermatitis, allergic encephalomyelitis, glomerulonephritis, Goodpasture's Syndrome, Graves'Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Autoimmune Pulmonary Inflammation, Guillain-Barre Syndrome, insulin dependent diabetes mellitis, and autoimmune inflammatory eye disease, naphylaxis, hypersensitivity to an antigenic molecule, or blood group incompatibility, graft versus host disease (GVHD), lupus erythematosus, viral/bacterial/parasitic infections, multiple sclerosis, autoimmune disease, allergies, immunodeficiencies, graft versus host disease, asthma, emphysema, ARDS, inflammation and modulation of the immune response, viral pathogenesis, aging-related disorders, Th1 inflammatory diseases such as rheumatoid arthritis, multiple sclerosis, inflammatory bowel diseases, AIDS, wound repair, heart attacks, heart failure, muscular dystrophy, bed sores, diabetic ulcers, oxidative damage, and tissue damage such as sinusitis or mucositis, wrinkles, eczema or dermatitis, dry skin, obesity, diabetes, endocrine disorders, anorexia, bulimia, renal artery stenosis, interstitial nephritis, glomerulonephritis, polycystic kidney disease, systemic, renal tubular acidosis, IgA nephropathy, nephrological disesases, hypercalceimia, Lesch-Nyhan syndrome, Von Hippel-Lindau (VHL) syndrome, trauma, regeneration (in vitro and in vivo), Hirschsprung's disease, Crohn's Disease, appendicitis, endometriosis, laryngitis, psoriasis, actinic keratosis, acne, hair growth/loss, allopecia, pigmentation disorders, myasthenia gravis, alpha-mannosidosis, beta-mannosidosis, other storage disorders, peroxisomal disorders such as Zellweger syndrome, infantile refsum disease, rhizomelic chondrodysplasia (chondrodysplasia punctata, rhizomelic), and hyperpipecolic acidemia, osteoporosis, muscle disorders, urinary retention, Albright Hereditary Ostoeodystrophy, ulcers, Alzheimer's disease, stroke, Parkinson's disease, Huntington's disease, cerebral palsy, epilepsy, Lesch-Nyhan syndrome, multiple sclerosis, ataxia-telangiectasia, behavioral disorders, addiction, anxiety, pain, neuroprotection, Stroke, Aphakia, neurodegenerative disorders, neurologic disorders, developmental defects, conditions associated with the role of GRK2 in brain and in the regulation of chemokine receptors, encephalomyelitis, anxiety, schizophrenia, manic depression, delirium, dementia, severe mental retardation and dyskinesias, Gilles de la Tourette syndrome, leukodystrophies, cancers, breast cancer, CNS cancer, colon cancer, gastric cancer, lung cancer, melanoma, ovarian cancer, pancreatic cancer, kidney cancer, colon cancer, prostate cancer, neuroblastoma, and cervical cancer, Neoplasm; adenocarcinoma, lymphoma; uterus cancer, benign prostatic hypertrophy, fertility, control of growth and development/differentiation related functions such as but not limited maturation, lactation and puberty, reproductive malfunction, and/or other pathologies and disorders of the like. Additional autoinflammatory disorders are cryopyrin-associated periodic syndrome (CAPS), such as Muckle-Wells syndrome (MWS), familial cold autoinflammatory syndrome (FCAS), or neonatal-onset multisystem inflammatory disease (NOMID); tumor necrosis factor receptor associated periodic syndrome (TRAPS); hyperimmunoglobulin D syndrome (HIDS)/mevalonate kinase deficiency (MKD); familial mediterranean fever (FMF); Behcet Disease; Pyoderma Gangraenosum; systemic onset of juvenile idiopathic arthritis (sJIA); Schnitzler syndrome; or Hidradenitis Suppurativa.

In a particular embodiment, alone or in combination with the previous particular embodiments, the autoinflammatory disorder is an interferonopathy. As used herein, the term "interferonopathy" refers to the medical condition or issue that are caused by, or exacerbated by, an increase or dysregulation of Type-I IFN. Examples of interferonopathies include, without limited to, systemic lupus erythematosus, Aicardi-Goutieres syndrome, Candle (chronic atypical neutrophilic dermatosis with lipodystrophy and elevated temperature), Savi (STING-associated vasculopathy with onset in infancy), Spencd (spondyloenchondro-dysplasia with immune dysregulation) or Singleton-Merten syndrome. In a more particular embodiment, the interferonopathy is selected from the group consisting of systemic lupus erythematosus, Aicardi-Goutieres syndrome, Candle, Savi, Spencd and Singleton-Merten syndrome.

In the present invention, the subject to be treated can be any animal. Preferably, the animal to be treated is a mammal, more preferably, a primate, still more preferably, a human of any sex, race or age.

In previous paragraphs, the different features which characterize the amino acid of the invention for use as a medicament, or for use in the treatment of auto-inflammatory disorders have been disclosed, and all the possible alternative or variants are included within the context of the present invention, with the proviso that the isolated amino acid sequence is not the sequence SEQ ID NO: 2, or SEQ ID NO: 3. In a particular embodiment, with the proviso that the isolated amino acid sequence of the invention for use as a medicament or for use in the treatment of auto-inflammatory disorders is not the sequence SEQ ID NO: 17 (amino acid sequence of Full B18 protein).
SEQ ID NO: 2 
MTMKMMVHIYFVSLLLLLFHSYAIDIENEITEFFNKMRDTLPAKDSKW
LNPAPPSCIPKTYELGTHDKYGIDLYCGILYAKHYNNITWYKDNKEINI
DDIKYSQTGKELIIHNPELEDSGRYDCYVHYDDVRIKNDIVVSRCKILTVIPS
QDHRFKLILDPKINVTIGEPANITCTAVSTSLLIDDVLIEWENPSG
WLIGFDFDVYSVLTSRGGITEATLYFENVTEEYIGNTYKCRGHNYYFE
KTLTTTVVLE
SEQ ID NO: 3 
MPPSCIPKTYELGTHDKYGIDLYCGILYAKHYNNITWYKDNKEINIDDI

KYSQTGKELIIHNPELEDSGRYDCYVHYDDVRIKNDIVVSRCKILTVIP

SQDHRFKLILDPKINVTIGEPANITCTAVSTSLLIDDVLIEWENPSGWLI

GFDFDVYSVLTSRGGITEATLYFENVTEEYIGNTYKCRGHNYYFEKTLTTTVVLE
SEQ ID NO: 17

MTMKMMVHIYFVSLLLLLFHSYAIDIENEITEFFNKMRDTLPAKDSKWLNPACMFGG
TMNDIAALGEPFSAKCPPIEDSLLSHRYKDYVVKWERLEKNRRRQVSNKRVKHGDLW
IANYTSKFSNRRYLCTVTTKNGDCVQGIVRSHIRKPPSCIPKTYELGTHDKYGIDLY
CGILYAKHYNNITWYKDNKEINIDDIKYSQTGKELIIHNPELEDSGRYDCYVHYDDV
RIKNDIVVSRCKILTVIPSQDHRFKLILDPKINVTIGEPANITCTAVSTSLLIDDVL
IEWENPSGWLIGFDFDVYSVLTSRGGITEATLYFENVTEEYIGNTYKCRGHNYYFEK
TLTTTVVLE

### Fusion protein of the invention

In the present description, the inventors have developed a new soluble receptor to be used in the clinic to block IFN-I. The new soluble receptor, a viral IFN-I decoy receptor, comprises, or consist of, (i) the D3 immunoglobulin-like domain from the B18 protein coming from vaccinia virus linked to (ii) a cell binding domain. This new soluble receptor not only retains the B18 full protein ability of binding IFN_I, but also it can be retained around the site of infection, enhancing the immunomodulatory activity of D3 and improving its therapeutic potential.

Therefore, in another aspect, the present invention relates to a fusion protein, hereinafter "fusion protein of the invention", comprising
(i) An amino acid sequence comprising, or consisting of, an amino acid sequence having a sequence identity of at least 70, 80, 90, 91, 92, 93, 94, 95, 96, 97, 98, or 99 % with the sequence SEQ ID NO: 1, and
(ii) an amino acid sequence comprising, or consisting of, a cell surface binding domain,
with the proviso that the fusion protein is not the sequence SEQ ID NO: 17 (B18 full sequence).

In a particular embodiment of the fusion protein of the invention, the amino acid sequences (i) and (ii) are heterologous to each other. As used herein, the term "heterologous" refers to the different origin of the amino acids sequences (i) and (ii), i.e.: they come from different molecules. In this case, the fusion protein of the invention is a chimeric protein.

The terms "sequence identity" and "cell surface binding domain" have been defined in the previous inventive aspect, as well as the amino acid sequence SEQ ID NO: 1.

In another particular embodiment, alone or in combination with the particular embodiment cited above, the amino acid sequence (i) comprises, or consists of, an amino acid sequence having a sequence identity of 100% with the sequence SEQ ID NO: 1.

In another particular embodiment, alone or in combination with the particular embodiments cited above, the cell surface binding domain is
(a) an amino acid sequence having a sequence identity of at least 70, 80, 90, 91, 92, 93, 94, 95, 96, 97, 98, 99 or 100% with the sequence SEQ ID NO: 4 (GAG-BD),
(b) an amino acid sequence having a sequence identity of at least 70, 80, 90, 91, 92, 93, 94, 95, 96, 97, 98, 99 or 100% with the sequence SEQ ID NO: 5 (full D1 from B18), or
(c) an amino acid sequence having a sequence identity of at least 70, 80, 90, 91, 92, 93, 94, 95, 96, 97, 98, 99 or 100% with the sequence SEQ ID NO: 6 (MC MC54L-GAG).

The sequences SEQ ID NO: 4, SEQ ID NO: 5 and SEQ ID NO: 6 have been defined in the previous inventive aspect.

In another particular embodiment, alone or in combination with all or each one of the previous particular embodiments, the isolated amino acid sequence comprises a linker between the sequence (i) and (ii). The term "linker" has been defined previously as well as examples of linkers which can be used in the present invention. Nevertheless, in a more particular embodiment, the linker used in the fusion protein of the invention comprises the sequence SEQ ID NO: 10.

In another particular embodiment, alone or in combination with each one of the previous particular embodiments, the fusion protein of the invention comprises the amino acid sequence SEQ ID NO: 11, SEQ ID NO: 13 or SEQ ID NO: 15.

In another aspect, the invention relates to an isolated polynucleotide, hereinafter "polynucleotide of the invention", comprising a nucleotide sequence encoding the fusion protein of the invention as defined above, with the proviso that the nucleotide sequence is not sequence SEQ ID NO: 18.
SEQ ID NO: 18

gacaattaacgatctttataatatatcgtatccacctaccaaagtatagttgtattt
ttctcatgcgatgtgtgtaaaaaaactgatattatataaatattttagtgccgtata
ataaagatgacgatgaaaatgatggtacatatatatttcgtatcattattgttattg
ctattccacagttacgccatagacatcgaaaatgaaatcacagaattcttcaataaa
atgagagatactctaccagctaaagactctaaatggttgaatccagcatgtatgttc

ggaggcacaatgaatgatatagccgctctaggagagccattcagcgcaaagtgtcct
cctattgaagacagtcttttatcgcacagatataaagactatgtggttaaatgggaa
aggctagaaaaaaatagacggcgacaggtttctaataaacgtgttaaacatggtgat
ttatggatagccaactatacatctaaattcagtaaccgtaggtatttgtgcaccgta
actacaaagaatggtgactgtgttcagggtatagttagatctcatattagaaaacct
ccttcatgcattccaaaaacatatgaactaggtactcatgataagtatggcatagac
ttatactgtggaattctttacgcaaaacattataataatataacttggtataaagat
aataaggaaattaatatcgacgacattaagtattcacaaacgggaaaggaattaatt
attcataatccagagttagaagatagcggaagatacgactgttacgttcattacgac
gacgttagaatcaagaatgatatcgtagtatcaagatgtaaaatacttacggttata
ccgtcacaagaccacaggtttaaactaatactagatccaaaaatcaacgtaacgata
ggagaacctgccaatataacatgcactgctgtgtcaacgtcattattgattgacgat
gtactgattgaatgggaaaatccatccggatggcttataggattcgattttgatgta
tactctgttttaactagtagaggcggtattaccgaggcgaccttgtactttgaaaat
gttactgaagaatatataggtaatacatataaatgtcgtggacacaactattatttt
gaaaaaacccttacaactacagtagtattggagtaaatatacaatgcatttttatat
acattactgaataattattattattatttatatcgtatttgtgctataacgcgacta
tctaggtatttgtatctcaccgatagagaacatataaat

Thus, the polynucleotide of the invention is the coding sequence of the fusion protein of the invention. The term "coding sequence" means a polynucleotide which directly specifies the amino acid sequence of a protein. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide. Likewise, as the skilled person in the art knows, the nucleotide sequences may be modified to be humanized.

In a particular embodiment, the nucleotide sequence encoding the fusion protein of the invention comprises, or consists of, the sequence SEQ ID NO: 12, SEQ ID NO: 14 or SEQ ID NO: 16. These sequences have been defined above for another inventive aspect, being said definitions applicable to the present inventive aspect.

The fusion protein of the invention may be of any length. Nevertheless, in a particular embodiment, alone or in combination with all or each one of the previous particular embodiments, the length of the fusion protein is from 100 to 400 amino acids, or from 100 to 350 amino acids, or from 100 to 300 amino acids, or from 100 to 250 amino acids or from 100 to 200 amino acids, or from 100 to 150 amino acids.

A polynucleotide sequence may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression. Thus, in another aspect, the present invention relates to a vector comprising, or consisting of, the nucleotide sequence encoding the fusion protein of the invention, hereinafter "vector of the invention".

As used herein, the term "vector" or "plasmid" refers to any vehicle used to transfer foreign genetic material into a cell. Said vector can be a viral vector or a non-viral vector, such as a plasmid. By way of a non-limiting illustration, said vectors can be viral vectors based on retroviruses, adenoviruses, alphaviruses, etc., or in case of non-viral vectors, the vectors can be DNA-liposome, DNA-polymer, DNA-polymer-liposome complexes, pSilencer 4.1-CMV (Ambion), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-1, pML2d, pTDTI, etc. Said viral and non-viral vectors comprising the polynucleotide encoding in its natural state a fluorescent protein can be administered to the cell by the conventional methods previously cited.

The vector can contain, among others, multiple cloning sites, expression regulating sequences, suitable replication origins for the host cell in which the vector is introduced, selection markers, etc. In a particular embodiment, the vector is an expression vector which allows the expression of the polynucleotide in the target cell and generally has a promoter sequence (or expression regulating sequences) that drives expression thereof. The promoter sequence preceding the polynucleotide is operatively bound to the said polynucleotide. As used in this description, the expression "operatively bound" means that the polynucleotide is within the correct reading frame for their expression under the control of said regulating sequences. The regulating sequences useful for the present invention can be nuclear promoting sequences or, alternatively, enhancer sequences and/or other regulating sequences increasing the expression of the polynucleotide. The promoter can be constitutive or inducible. If the constant expression of the polynucleotide is desired, then a constitutive promoter is used. Examples of well-known constitutive promoters include the cytomegalovirus (CMV) immediate-early promoter, the Rous sarcoma virus promoter, and the like. A number of other examples of constitutive promoters are well known in the art and can be used in implementing the invention. If the controlled expression of the polynucleotide is desired, then an inducible promoter must be used. In a non-induced state, the inducible promoter must be "silent". "Silent" is understood to mean that in the absence of an inducer, little or no expression of the polynucleotide is detected; in the presence of an inducer, however, the expression of the polynucletide occurs. The expression level can frequently be controlled by varying the concentration of the inducer so that the inducible promoter is stimulated more strongly or weakly and consequently, the concentration of the polynucletide transcribed product is affected. Examples of well-known inducible promoters are: an androgen or estrogen-responsive promoter, a doxycicline-responsive promoter, a metallothionein promoter, or a promoter responding to ecdysone. Other various examples are well known in the art and can be used for implementing the invention.

In order to express the polynucleotide encoding the fusion protein of the invention, the nucleotide sequence encoding thereof has to be introduced in an expression system such as a host cell. Thus, the nucleotide sequence may be optimized for a proper expression in the chosen host cell. Thus, in another aspect, the present invention relates to a cell comprising, or consisting of, the polynucleotide of the invention, hereinafter "cell or host cell of the invention".

The term "host cell" means any cell type that is susceptible to transformation, transfection, transduction, and the like with a nucleic acid construct or expression vector comprising the polynucleotide of the present invention.

The present invention also relates to host cells, comprising the isolated polynucleotide of the invention, which are advantageously used in the recombinant production of the fusion protein. A vector is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication.

The host cell may be any cell useful in the recombinant production of a fusion protein of the present invention, e.g., a prokaryote or a eukaryote such as a mammalian, insect, plant, or fungal cell.

The prokaryotic host cell may be any Gram positive bacterium or a Gram negative bacterium. Gram positive bacteria include, but not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but not limited to, *E. coli, Pseudomonas, Salmonella, Campylobacter, Helicobacter, Flavobacterium, Fusobacterium,* I*lyobacter, Neisseria,* and *Ureaplasma.* In a particular embodiment, the host cell is E. *coli.* Examples of E. *coli* strains useful for vector propagation and cloning procedures include, without limiting to, DH10B (Invitrogen), DH5α (ATCC, Invitrogene), DM1 (Invitrogen), GeneHogs (Invitrogen), HB101 (Bio-Rad, Invitrogene, Promega), INV110 (Invitrogen), JM109 (ATCC, Promega), JS5 (Bio-Rad), LE392 (ATCC), NM522 (AS), SCS110 (Stratagene), STBL4 (Invitrogen), SURE (ATCC, Stratagene), TG1 (Stratagene), XL10-Gold (Stratagene) and XL1-Blue MRF (Stratagene). In a particular embodiment, the E. *coli* strain for cloning and storage is E. *coli* DH10B. In another particular embodiment, the *E. coli* strain for expressing the polynucleotide of the invention is *E. coli* BL21 (DE3) (EMD Biosciences, San Diego, CA).

Examples of insect cells include, without limiting to, *Spodoptera frugiperda* cells (such as SF9 cell or SF21 cell or Sf900+), a BTI-Tn-5B1-4 insect cell from Trichoplusia ni (High-five^{™}, Invitrogen, Carlsbad Calif.), expresSF+^{®}, Drosophila Schneider 2 (S2) Cells, Se301, SeIZD2109, SeUCR1, MG-1, Tn368, HzAm1, Ha2302, Hz2E5 and High Five from Invitrogen. In the context of the present invention, not only insect cells can be used for producing the fusion protein of the invention, but also whole insect itself such as a larva.

Examples of the fungi host cells includes, without limiting to, Aspergillus (e.g. *Aspergillus awamori, Aspergillus fumigatus, Aspergillus foetidus, Aspergillus japonicus, Aspergillus nidulans, Aspergillus niger* or *Aspergillus oryzae* cell), Aureobasidium, Bjerkandera, Ceriporiopsis (e.g. *Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora),* Chrysosporium (e.g. *Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum*)*,* Coprinus (e.g. *Coprinus cinereus*)*,* Coriolus (e.g. *Coriolus hirsutus*)*,* Cryptococcus, Filibasidium, Fusarium (e.g. *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell), Humicola (e.g. *Humicola insolens, Humicola lanuginosa*)*,* Magnaporthe, Mucor (e.g. *Mucor miehei*)*,* Myceliophthora (e.g. *Myceliophthora thermophila*)*,* Neocallimastix, Neurospora (e.g. *Neurospora crassa*)*,* Paecilomyces, Penicillium (e.g. *Penicillium purpurogenum*)*,* Phanerochaete (e.g. *Phanerochaete chrysosporium*)*,* Phlebia (e.g. *Phlebia radiata*)*,* Piromyces, Pleurotus (e.g. *Pleurotus eryngii*)*,* Schizophyllum, Talaromyces, Thermoascus, Thielavia (e.g. *Thielavia terrestris*)*,* Tolypocladium, Trametes (e.g. *Trametes villosa or Trametes versicolor*)*,* or Trichoderma (e.g. *Trichoderma harzianum, Trichoderma koningii, Trichoderma longibrachiatum, Trichoderma reesei, or Trichoderma viride* cell) cell.

The host cell may also be a yeast cell. "Yeast" as used herein includes ascosporogenous yeast (Endomycetales), basidiosporogenous yeast, and yeast belonging to the Fungi Imperfecti (Blastomycetes). More preferably, the yeast host cell is a Candida, Hansenula, Kluyveromyces (e.g. *Kluyveromyces lactis*)*,* Pichia (e.g. *Pichia pastoris*)*,* Saccharomyces (e.g. *Saccharomyces carlsbergensis, Saccharomyces cerevisiae, Saccharomyces diastaticus, Saccharomyces douglasii, Saccharomyces kluyveri, Saccharomyces norbensis, or Saccharomyces oviformis,* etc.), Schizosaccharomyces, or Yarrowia cell (*Yarrowia lipolytica*)*.*

The host cell may also be microalgae. Examples of microalgae include, without limiting to, microalgae from the genera *Chlamydomonas* (e.g. *Chlamydomonas reinhardtii*), *Botryococcus, Neochloris, Nannochloropsis, Phorphyridium, Scenedesmus, Chlorella, Tetraselmis* and *Spirulina.* The chloroplast of these algae are particularly well suited to the production of bacterial proteins as it mimics the native bacterial expression environment of these proteins while being devoid of their cell wall target.

The host cell may also be a plant cell, wherein the vacuolar deposition of recombinant proteins in plant vegetative organs is used to increase yields. To achieve this, the polynucleotide of the invention may be fused to sequence specific vacuolar sorting signals (ssVSS) typical of proteases or proteinase inhibitors and/or Ct-VSS representative of storage proteins or plant lectins. Both types of motifs were capable to increase accumulation. Importantly, the type of VSSs or position, either the N or C-terminus, did not alter protein stability, levels or post-translational modifications. Examples of plant cells include, without limiting to, *Nicotiana* sp. (e.g. *N. tabacum, N. benthamiana,* etc.) sugarcane, tomato (*Solanum lycopersicum*) and carrot (*Daucus carota*)*.*

The introduction of DNA into a cell may, for instance, be effected by protoplast transformation, by using competent cells, by electroporation or by conjugation. Any method known in the art for introducing DNA into a host cell can be used.

In another aspect, the present invention relates to a composition, hereinafter "composition of the invention", comprising the fusion protein, the polynucleotide, the vector and/or the cell of the invention.

According to the conventional techniques known to those skilled in the art, the composition according to the present invention may be formulated with excipient and/or carrier. Thus, in a particular embodiment, the composition of the invention comprises an excipient and/or carrier. In case of a therapeutic used of the composition of the invention, this may be formulated with a pharmaceutically acceptable excipient and/or carrier.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The term "carrier" refers to a compound which facilitates the incorporation of other compounds to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency. When the form of presentation is liquid, the carrier is the diluent. In a particular embodiment, the carrier is pharmaceutically acceptable.

The fusion protein of the invention may be employed as a therapeutic agent to be administered to a subject. In this case, the composition provided by this invention is considered a pharmaceutical composition which may be administered by any appropriate administration route; to this end, said composition will be formulated in the suitable form for the selected administration route.

The "galenic form" or "pharmaceutical form" is the arrangement to which the active ingredients and excipients adapt in order to form a medicament. It is defined by the combination of the form in which the pharmaceutical composition is presented by the manufacturer and the form in which it is administered.

Routes of administration include topical, ocular, nasal, pulmonary, buccal, parenteral (intravenous, subcutaneous, and intramuscular), oral, vaginal and rectal. Administration from implants is also possible. The composition of the invention, in particular the pharmaceutical composition, may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated.

The composition of the invention may also be administered directly into the blood stream, into muscle, or into an internal organ. Suitable means for parenteral administration include intravenous, intra-arterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The composition of the invention may also be administered intranasally or orally. Dosage forms for oral administration include, without limiting to, tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms. The pharmaceutical composition may also be provided in bandages or in sutures or the like.

Biodegradable particles (nanoparticles or microparticles) have been shown to be capable of delivering a variety of therapeutic agents including molecules such as polypeptides or proteins. Thus, the present invention also encompasses biodegradable microparticles o nanoparticles comprising the fusion protein, the polynucleotide, the vector, the cell or the composition of the invention.

The composition of the invention may comprise, further to the chimeric protein, the polynucleotide, the vector, or the cell of the invention, other therapeutic agents such as antibiotics, painkillers, etc., or other therapeutic agents useful in the treatment of an autoinflammatory disease. Examples of antibiotics include, without limited to, vancomycin, teicoplanin, oxacillin, flucloxacillin, dicloxacillin, first generation cephalosporins (such as cefazolin, cephalothin, cephalexin, etc.), lincosamides (such as clindamycin, lincomycin, etc.), cotrimoxazole, erythromycin, rifampicin, fusidic acid, linezolid, quinupristin/dalfopristin and any combination thereof.

In another aspect, the present invention relates to the fusion protein of the invention, the polynucleotide of the invention, the vector of the invention, the cell of the invention and/or the composition of the invention, for use as a medicament, preferably, for use in the treatment of an autoinflammatory disorder.

The terms "treatment" and "autoinflammatory disorder" have been defined previously, and said definitions are applicable to the present inventive aspect. Examples of autoinflammatory disorders have been mentioned above.

In a particular embodiment, the autoinflammatory disorder is an interferonopathy. The term "interferonopathy" has been defined previously. Examples of interferonopathies include, without limited to, systemic lupus erythematosus, Aicardi-Goutieres syndrome, Candle (chronic atypical neutrophilic dermatosis with lipodystrophy and elevated temperature), Savi (STING-associated vasculopathy with onset in infancy), Spencd (spondyloenchondro-dysplasia with immune dysregulation) or Singleton-Merten syndrome. In a more particular embodiment, the interferonopathy is selected from the group consisting of systemic lupus erythematosus, Aicardi-Goutieres syndrome, Candle, Savi, Spencd, and Singleton-Merten syndrome.

In the present invention, the subject to be treated can be any animal. Preferably, the animal to be treated is a mammal, more preferably, a primate, still more preferably, a human of any sex, race or age.

### DESCRIPTION OF THE DRAWINGS

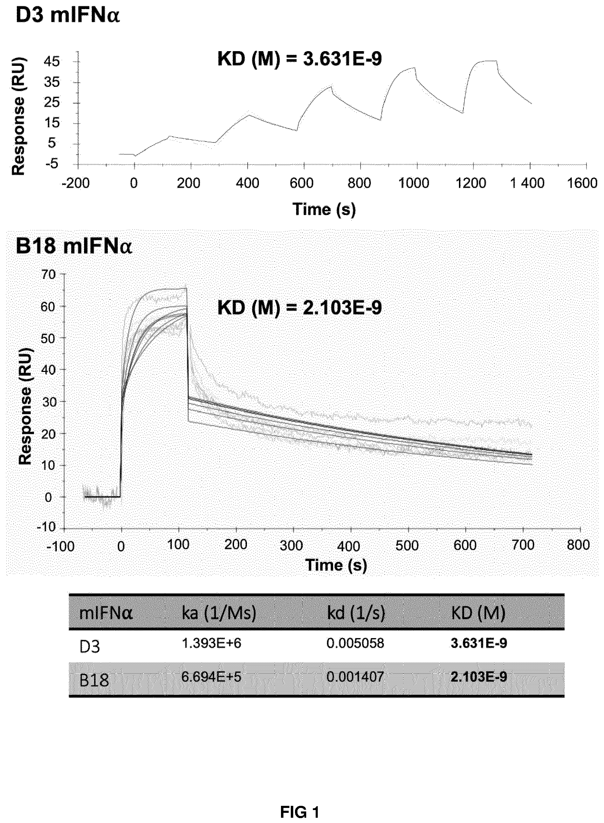

**Figure 1****. D3 binds IFN-I with high affinity.** SPR sensograms and fitting obtained for the determination of the kinetic constants of the interaction of D3 and B18 recombinant proteins expressed in bacteria with mIFN. D3 and B18 were immobilized in a SPR Biacore SA sensor chip and binding and dissociation of several concentrations of mIFN at 30µl/minute were recorded and adjusted to a 1:1 Langmuir fitting (solid lines). Kinetic parameters and calculated affinity constants of D3 and B18 are shown in the inset table.

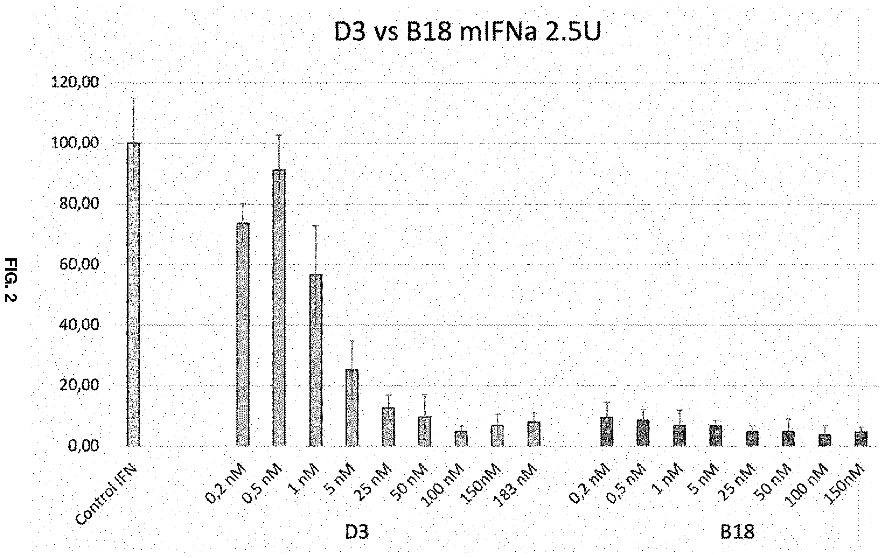

**Figure 2****. Determination of D3 protective capacity.** Increasing amounts of recombinant D3 and B18 were incubated at 37ºC with 2.5U of mIFN for 1 hour and then added to L929 cells. After 16h incubation, cells were infected with VSV, and cell viability was measured at 48 hpi. Data are means showing standard deviations of triplicates.
**Figure 3****. Schematic representation of the proposed modified D3 versions.** The origin of the different protein motives fused to D3 is shown, being GAG-BD and D1 from vaccinia virus B18 protein and MC-GAG-BD from molluscum contagiosum virus MC54L protein.

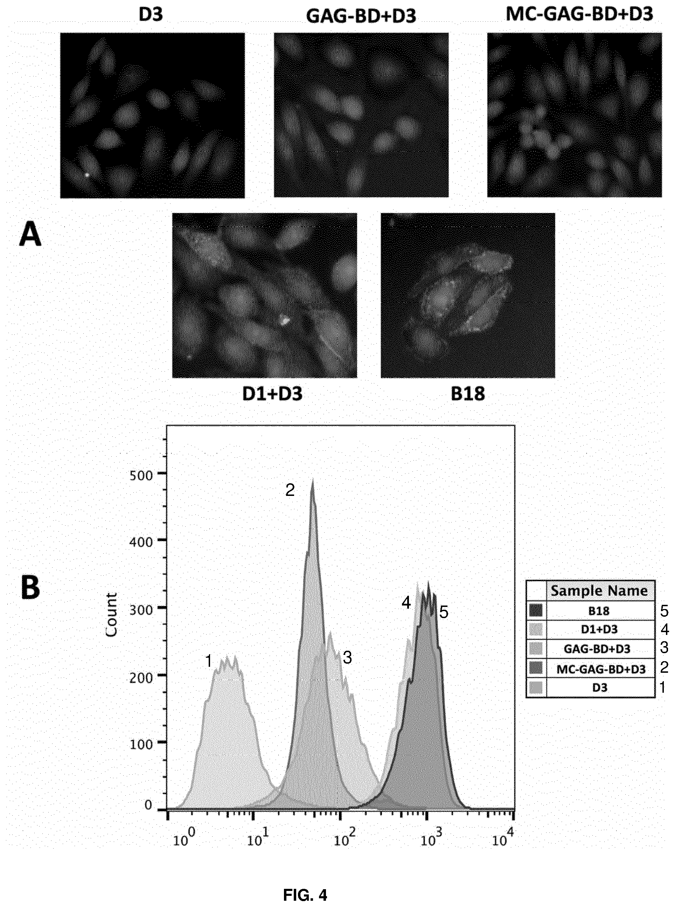

**Figure 4****. Cell surface binding assays.** A CHOK1 cells were incubated at 4 °C for 30 min with V5-tagged recombinant proteins. Proteins were detected by immunofluorescence with an anti-V5 antibody and cell nuclei were stained with DAPI. B CHO-K1 cells were incubated at 4ºC with recombinant tagged proteins. Cell surface binding was assessed by flow cytometry using an anti-V5 antibody. In every case, experiments were performed in triplicate and a representative histogram is shown.

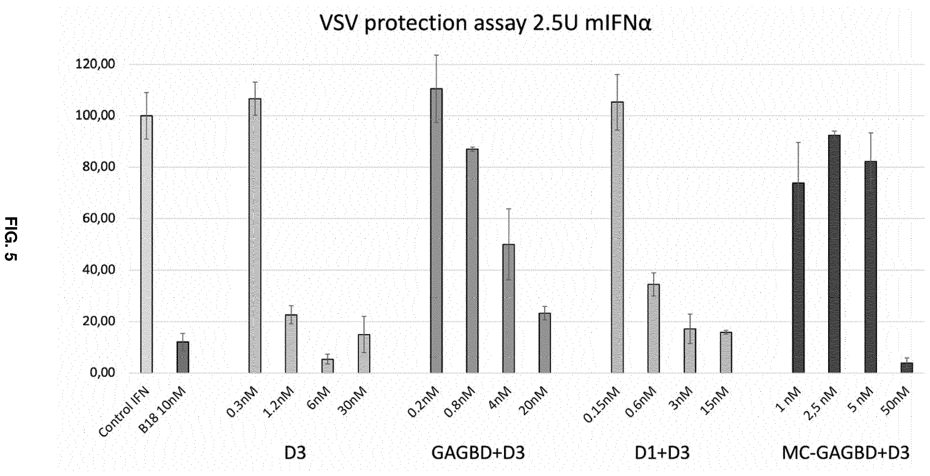

**Figure 5****. Determination of D3 versions protective capacity.** Increasing amounts of recombinant proteins were incubated at 37ºC with 2.5U of mIFN for 1 hour and then added to L929 cells. After 16 hours incubation, cells were infected with VSV, and cell viability was measured at 48 hpi. Data are means showing standard deviations of triplicates.

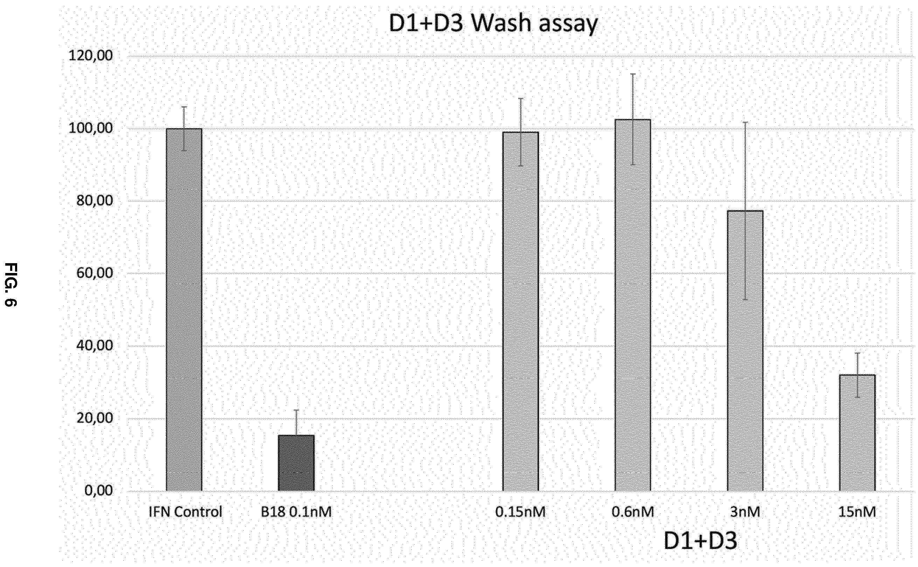

**Figure 6****. D1+D3 wash assay.** Increasing amounts of recombinant proteins were incubated at 37ºC with L929 cells for an hour and then extensively washed prior to addition of 2.5U mIFN. After 16h incubation, cells were infected with VSV, and cell viability was measured at 48 hpi. Data are means showing standard deviations of triplicates

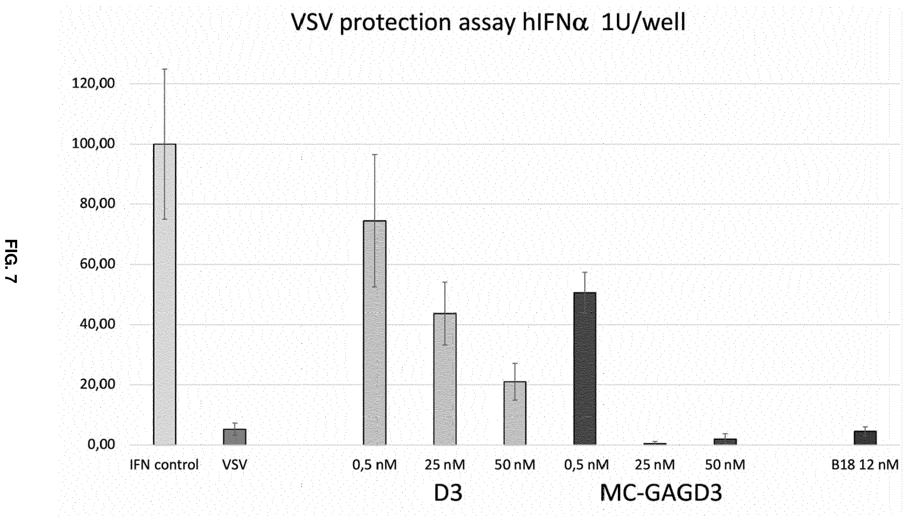

**Figure** 7. **Determination of protective capacity against hIFNα.** Increasing amounts of recombinant proteins were incubated at 37ºC with 1U of human IFNα for 1 hour and then added to HeLa cells. After 16h incubation, cells were infected with VSV, and cell viability was measured at 48 hpi. Data are means showing standard deviations of triplicates.

### Examples

### MATERIALS AND METHODS

### Cell and viruses

The generation and amplification of baculovirus stocks were developed in Hi5 insect cells (BTI-Tn-5B1-4, Thermofisher). Hi5 cell monolayers were maintained at 28ºC and 80% humidity with TC-100 (Gibco) medium containing fetal bovine serum (FBS) 10%, 2 mM glutamine (Sigma) and an antibiotic mix (100 µg/mL gentamicin, 200 U/mL penicillin and 20 µg/mL streptomycin). Cells were subcultured 1:10 each 4 days to ensure 100% confluence is not reached. Suspension culture was maintained in shake flasks at 131 rpm and 28 ºC in Express Five medium (Gibco) supplemented with an antibiotic mix (100 µg/mL gentamicin, 200 U/mL penicillin and 20 µg/mL streptomycin). Cells were diluted to 0.5×10⁶/mL each two days to ensure a concentration of more than 4×10⁶/mL is not reached.

L929 cells (ATCC CCL-1) were maintained in Dulbecco's modified Eagle's medium (DMEM) containing 10% FBS medium supplemented with 2 mM glutamine (Sigma) and antibiotics (100 µg/mL gentamicin, 200 U/mL penicillin and 20 µg/mL streptomycin) at 37ºC, 95% relative humidity and 5% CO₂. Cells were subcultured 1:10 each 4 days to ensure 100% confluence is not reached.

HeLa cells (ATCC CCL-2) were maintained in Dulbecco's modified Eagle's medium (DMEM) containing 10% FBS medium supplemented with 2 mM glutamine (Sigma) and antibiotics (100 µg/mL gentamicin, 200 U/mL penicillin and 20 µg/mL streptomycin) at 37ºC, 95% relative humidity and 5% CO₂. Cells were subcultured 1:10 each 3 days to ensure 100% confluence is not reached.

Chinese hamster ovary K1 (CHO-K1, ATCC CCL-61) cells were grown in 10% FBS containing DMEM-Ham's F12 (1:1) medium supplemented with 2 mM glutamine and antibiotics mix (100 µg/mL gentamicin, 200 U/mL penicillin and 20 µg/mL streptomycin) at 37ºC, 95% relative humidity and 5% CO₂. Cells were subcultured 1:10 each 3 days to ensure 100% confluence is not reached.

Vesicular stomatitis virus (VSV) strain Cocal was obtained from Dr. William James (Sir William Dunn School of Pathology, Oxford University, Oxford, UK). VSV plaque assays were carried out in Vero cells (ATCC CCL-81).

### Cloning of sequences

B18 coding sequence from VACV-Western Reserve (GenBank: D01019.1 SEQ ID NO: 18) was amplified by PCR using high fidelity Vent DNA polymerase (New England Biolabs) from previously described plasmid pMF2 (Fernández de Marco et al., 2010). DNA fragments were purified with PCR purification kit (Quiagen), digested with restriction enzymes (New England Biolabs) and subsequently cloned in BamHI and Notl sites in pAL7, a modified pFastBac1 vector bearing the honeybee melittin signal peptide at the 5' region and C-terminal V5 and 6xHis tags used for detection and purification of the recombinant protein. A similar procedure was used to obtain the molluscum contagiosum virus GAG-binding motif coding sequence. For expression in bacteria, coding sequences were similarly amplified by PCR and then cloned in Ncol and Xhol sites into commercial plasmid pET28b (Novagen) bearing a C-terminal 6x-his tag. The oligonucleotides and the restriction enzyme used are summarized in the following table (Table 1):

**Table 1. Oligonucleotides and restriction enzymes used in the present invention.**

| **Protein** | **Oligo** | **Sequence** | **Restriction enzyme** | **Destination vector** |
|---|---|---|---|---|
| B18 (bacteria) | ASDfor_1 | gcgCCATGGgcca cagttacgccatagaca tcg (SEQ ID NO: 19) | Ncol | pAS1 |
| B18 (bacteria) | ASDrev_1 | cgcCTCGAGctcca atactactgtagttgtaa g (SEQ ID NO: 20) | Xhol | pAS1 |
| D3 (bacteria) | ASDfor_3 | cgcCCATGGgccc gtcacaagaccacagg tttaaac (SEQ ID NO: 21) | Ncol | pAS3 |
| D3 (bacteria) | ASDrev_3 | cgcCTCGAGctcca atactactgtagttgtaa g (SEQ ID NO: 22) | Xhol | pAS3 |
| D3 | JA_D3F_3 | gcgGGATCCcaca ggtttaaactaatacta gatcc (SEQ ID NO: 23) | BamHI | pJA7 |
| D3 | JA_D3_2 | GCGGCCGCtctc caatactactgtagttgt aaggg (SEQ ID NO: 24) | Notl | pJA7 |
| GAGBD-D3 | JA_Sticker_F 1 | gcgccatggcaGGA TCCaaatggg (SEQ ID NO: 25) | BamHI | pJA8 |
| GAGBD-D3 | JA_Sticker_R 1 | gcggaattcGTCGA Cggaaccacc (SEQ ID NO: 26) | SaII | pJA8 |
| MCGAGBD-D3 | JA_MCGAG BD_F_3 | cgcGGATCCgcac agccgccggg (SEQ ID NO: 27) | BamHI | pJA10 |
| MCGAGBD-D3 | JA_MCGAG BD_R_3 | cgcGTCGACcctg acatgtcg (SEQ ID NO: 28) | Sall | pJA10 |
| D1-D3 | JA_D1_F | gcgGGATCCatag acatcgaaaatg (SEQ ID NO: 29) | BamHI | pJA9 |
| D1-D3 | JA_D1_R | cgcGTCGACttttct aatatgagatctaacta taccc (SEQ ID NO: 30) | Sall | pJA9 |

Plasmids were transformed in *E.coli* DH5α by 60s heat shock at 42ºC and then checked by restriction digestion. Finally, plasmids were sequenced to confirm the presence of the desired sequences and the absence of inadvertent mutations and were maintained in *E.coli* DH5α frozen at -80ºC in LB 15% glycerol.

### Generation of recombinant baculoviruses

The recombinant baculovirus expressing the VACV IFNα/βBP (B18) has been previously described (Fernández de Marco et al., 2010. The FASEB Journal, 24(5), 1479-1488). New recombinant baculoviruses were obtained using the Bac-to-Bac expression system (Invitrogen) following the manufacturer's instructions. Briefly, cloned genes in pFastBac1 can integrate into bacmids by transformation of competent DH10bac cells (Invitrogen). Recombinant bacmids were purified and transfected in Hi5 cells using lipofectamine 2000 (Invitrogen) according to the manufacturer's instructions. The resulting recombinant baculoviruses were collected from supernatants at 72 hours post transfection and maintained at 4ºC in the dark. Baculovirus stocks were amplified by infection of Hi5 cells at low multiplicity of infection (moi = 0.01-0.05 pfu/cell) in two consecutive cycles, generating high titer stocks for recombinant protein expression.

### Expression and purification of recombinant proteins.

For the expression of B18 and derived proteins in bacteria, we adapted a previously described protocol (Kim et al., 2017. PloS One, 12(12), e0189308). *E*. *coli* (BL21 strain) were transformed with recombinant pAS1 and pAS3 and incubated in 40 mL of LB (Sigma) medium supplemented with kanamycin (50 m/mL) for 24 hours at 37ºC and shaking (220 rpm). Then, inoculum was diluted 1:25 in LB and incubated at 37ºC and shaking (220 rpm) until an optic density at 600 nm of 0.6-0.8 is reached. At that point protein expression was induced by addition of 1 mM IPTG and bacteria were incubated for another 6 hours in the same conditions. Next, bacteria were collected by centrifugation (5400g, 20 minutes), washed with water and resuspended in lysis buffer (20% sucrose, 20mM HEPES (Sigma) pH 7.5, 5 mM MgCl₂ (Sigma), 0.1% Triton X-100 (Sigma), 1 mg/mL lysozyme (Sigma), 1 µg/mL DNAse I (Sigma) and 0.2 mM PMSF (Sigma). After 1 hour incubation at RT, 0.5% sodic deoxicolate (SDC, Sigma) was added and lysis was completed by sonication (20 pulses 30s each with 1minute breaks on ice) using Labsonic M. Inclusion bodies were isolated by 30 minutes centrifugation at 13,000g 4ºC and washed once with 100 mM Tris pH 8.0, 2 mM EDTA, 0.5% SDC and twice with TE buffer (100 mM Tris pH 8.0, 2 mM EDTA, Sigma), then frozen at -80 ºC. Inclusion bodies containing recombinant proteins were solubilized for 2 hours at RT in buffer 50 mM Tris pH 10 containing 2% sodium lauroyl sarcosinate (Sigma) and oxidized for 24 hours by adding 100 µM CuSO4 (Sigma). Finally, protein was precipitated by centrifugation (9500g, 20 minutes) with 40 % (NH4)2SO4 (m/v, Sigma)) and dissolved in 20 mM Na2HPO4 pH 7.4, 500 mM NaCl, 20 mM imidazole and 6 M urea.

For the expression in baculovirus, supernatants from Hi5 cells infected at high moi (2-5 pfu/cell) with the corresponding baculovirus were collected at 72 hours postinfection. First, supernatants were clarified by centrifugation for 5 minutes at 500g and then concentrated to 2.5 mL by ultrafiltration using the Stirred Ultrafiltration Cell 8200system (Amicon) and YM membranes (Millipore). Next, they were buffer exchanged against 50 mM phosphate, 300 mM NaCl and 10 mM imidazole, pH 7.4 using gel filtration columns PD10 (Amersham Biosciences).

For purification, protein extracts from either baculovirus or bacteria were incubated with 0.6 mL of Ni-NTA agarose resin (Qiagen) for 1 hour at 4ºC, mounted onto a column (Poly-Prep^{®}, Bio-Rad), washed with 40 mL washing buffer ((50 mM sodic phosphate (pH 7.4), 300 mM NaCl, 20 mM imidazole) and eluted with increasing concentrations of imidazole to perform affinity-purification of recombinant proteins. Protein was eluted in 0.6 mL fractions that were then analyzed by Coomassie blue-stained SDS-PAGE. Selected fractions with the best purity and quantity of protein were concentrated using Vivaspin 500 (Sartorius) microcolumns. Finally, protein purity and quantity were analyzed on Coomassie blue-stained SDS-PAGE and quantified by gel densitometry using BSA (Sigma) calibration.

### Surface Plasmon Resonance assays

To determine affinity constants, we conducted SPR assays using a Biacore X100 (Cytiva). 340 Response Units of each protein were immobilized in the flow cell number 2 of a NTA chip (Cytiva), using the 6xHis tag followed by amine coupling and leaving flow cell number 1 free to use as reference. Next, increasing concentrations of murine IFN-I alpha subtype A (PBL-Interferon source) were injected at 30 µL/minute in HBS-EP (Cytiva). Binding curves were fitted according to a 1:1 Langmuir model and analyzed using BiaEvaluation 3.2 software (Cytiva).

### Surface binding assays by flow cytometry.

CHO-K1 cells were detached with 4 mM EDTA at 37 °C (to maintain GAGs integrity) and harvested in PBS. Cells (3×10⁵ per experimental point) were incubated for 30 minutes on ice with 250 nM of the indicated viral recombinant proteins in 50 µL PBS-staining (supplemented with 10% FCS and 1% BSA). Cells were then extensively washed with FACS buffer (PBS, 0.01% sodium azide and 0.5% bovine serum albumin) and incubated for 45 minutes at 4 ºC with monoclonal anti-V5 antibody (Invitrogen) diluted 1:500 followed by anti-mouse IgG-A488 (Molecular Probes) diluted 1:500 in PBS for 45 minutes in the dark. Finally, cells were resuspended in 0.5 mL FACS buffer and analyzed in a FACSCalibur flow cytometer (BD Sciences). Data was later analyzed with FlowJo 7.2.2 (Treestar).

### Surface binding assays by immunofluorescence.

CHO-K1 cells seeded onto glass coverslips in 24-well plates were incubated with 250 nM of purified recombinant viral proteins for 30 minutes at 4 °C. Cells were then extensively washed with ice-cold PBS and fixed with 4% paraformaldehyde in PBS for 12 minutes at room temperature. The membrane permeabilization step was omitted, thus after aldehyde quenching with 50 mM NH₄Cl for 5 minutes, cells were incubated with a monoclonal anti-V5 antibody diluted 1:500 (Invitrogen) followed by anti-mouse IgG-A488 (Molecular Probes) diluted 1:1000. Cell nuclei were stained with DAPI (Calbiochem). Samples were finally washed with water and ethanol and mounted on slides using Prolong mount media (Invitrogen). Images were acquired in a Leica DMI6000B automated inverted microscope equipped with a Hamamatsu Orca R2 digital camera

### Neutralization of IFN-I antiviral activity assay

2.5 units of recombinant murine IFN-α subtype A (mIFNaA, PBL Assay Science) or 1 unit of recombinant human IFN-α (hIFNα) were incubated with purified viral recombinant proteins for 1 hour at 37 °C and then added to L929 or HeLa cells seeded in 96-well plates the day before (3000 cells/well). In the washing assay, recombinant proteins were added to cells, incubated for 60 minutes at 37 °C, and then extensively washed prior to the addition of IFN-I. In both cases, 16 hours after IFN-I addition, cells were washed and infected with VSV (moi 50 pfu/cell) and cell viability was determined at 48 hpi using the Cell Titer 96 Aqueous One Solution cell proliferation assay (Promega) following manufacturer indications. mIFNα and hIFNα were obtained from PBL-Interferon source.

### RESULTS

### D3 binds IFN-I with high affinity

To test whether D3 (SEQ ID NO: 1) is able to bind IFN-I on its own, 6xHis tagged versions of both B18 and D3 were expressed in bacteria and assessed their interaction with IFN-I by SPR using a Biacore X100 (Figure 1). Shockingly, despite being only a portion of less than 12 kDa from the full protein, the determined affinity for D3 was just a bit lower than the obtained for the complete B18, both in the nanomolar range. Both affinities were about 1 log lower than the affinity previously reported for B18 expressed in the baculovirus system (Fernández de Marco et al., 2010 (cited above); Montanuy et al., 2011. The FASEB Journal, 25(6), 1960-1971), likely due to structural differences related to the different expression systems used. We conclude that domain D3 retains the B18 ability to bind IFN-I with high affinity.

### D3 prevents IFN-I biological action

Next, the ability of D3 to block the IFN-I induced antiviral response in vitro was assessed (Figure 2). For this purpose, we developed previously described VSV protection assays (Hernáez et al., 2018. Nature Communications, 9(1), 5440; Montanuy et al., 2011 (cited above)). As expected, IFN-I addition protected cells from VSV infection. In contrast, preincubation of IFN-I with increasing amounts of D3 reduced the protective effects of IFN-I and cells died in a dose-dependent manner after VSV challenge, similarly to observed after preincubation with B18. However, to obtain full IFN neutralization the amounts of protein D3 were higher (about 1 log) than B18.

### Addition of GAG-binding motives confers cell surface binding ability to D3.

In order to transfer the cell surface binding property to D3, the addition of three different protein motives known to bind GAGs was proposed: i) the minimal GAG-binding domain (GAG-BD, SEQ ID NO: 4) described for B18 (GAG-BD+D3, SEQ ID NO: 11), ii) the full D1 from B18 (SEQ ID NO: 5) (D1+D3, SEQ ID NO: 13), containing the previous GAG-BD, (Montanuy et al., 2011 (cited above)) and iii) the GAG-BD from molluscum contagiosum virus (MC) IL-18 binding protein MC54L (SEQ ID NO: 6) (MC-GAG-BD+D3, SEQ ID NO: 15) (Figure 3). All of the protein motives were fused using a GGGGSx3 (SEQ ID NO: 10) flexible linker (Chen et al., 2013. Advanced Drug Delivery Reviews, 65(10), 1357-1369) in order to keep domains apart to help them fold properly and allow for some movement.

To test whether these modifications confer surface binding ability, diverse D3 V5-6xHis tagged versions fused to these protein motives were expressed and purified in the baculovirus system. Equal amounts of GAG-BD+D3 (), MC-GAG-BD+D3 () and D1+D3 () were incubated with CHO-K1 cells and binding was determined by immunofluorescence and flow cytometry using anti-V5 antibodies (Figure 4). We included the original D3 and B18 proteins as negative and positive controls, respectively, for surface binding. Examination of immunofluorescence revealed a subtle membrane pattern in GAG-BD+D3 and MC-GAG-BD+D3, indicating partial binding compared to B18. Interestingly, the D1+D3 membrane pattern was indistinguishable from that of B18, indicating that this protein retained most of the B18 cell binding ability. Flow cytometry analysis in CHO-K1 cells confirmed these results showing that both, GAG-BD+D3 and MC-GAG-BD+D3, have intermediate binding capacities while D1+D3 performed very similar to full B18. Together, these results indicated that addition of different GAG-binding motives was able to confer a spectrum of affinities for the cellular surface.

### Modified D3 versions maintain their ability to block IFN-I.

After testing their ability to bind to the cellular surface, the ability of the different D3 versions to block IFN-I in vitro was determined. In a similar VSV protection assay, preincubation of IFN-I with all the D3 versions reduced the protective effects of IFN-I in a dose-dependent manner, although different concentrations of proteins were required in each case to achieve complete protection (Figure 5). D1+D3 required less protein to block IFN-I, while MC-GAG-BD+D3 required the most. These disparities are likely due to differences in the specific affinities of each D3 version for mIFN.

After that, the ability to block IFN-I while bound to cell surface was tested using a modified version of the VSV protection assay, in which cells are incubated with proteins for 1 hour and then extensively washed prior to addition of IFN-I (Figure 6). While D3, GAG-BD+D3 and MC-GAG-BD+D3 were not able to protect in these conditions (data not shown), D1+D3 retained B18 capacities and was able to reduce the effects of IFN-I in a dose-dependent manner. Although it may be possible that GAG-BD+D3 and MC-GAG-BD+D3 were not able to bind IFN-I while bound to the cell surface, we think that they were simply washed away because their surface binding is not as tight as that of B18. These results prove that D1+D3 retains both functions from B18 and is able to develop them at the same time, mimicking the original protein.

Finally, the ability of the D3 versions to bind hIFNα and block its biological action in a new VSV protection assay was tested (Figure 7). In this case, some of the D3 versions (D3 and MC-GAG-BD+D3) were able to reduce the protective effects of hIFN-I in a dose-dependent manner. Interestingly, MC-GAG-BD+D3, the construction that needed the highest concentration to inhibit murine IFN-I, was the most effective at inhibiting human IFN-I. Although the concentration of proteins required for protection in this assay were generally higher than for murine IFN-I, this is influenced by the different cells lines and interferons used.

### DISCUSSION

The unsuccessful current therapies for interferonopathies, including monoclonal antibodies against IFN-I and IFNAR, call for research in new molecules that can block IFN-I. Viruses have optimized their immunomodulatory mechanisms during millions of years of evolution with their hosts and may now serve as an inspiration to fix this problem.

The present invention shows that D3 from VACV B18 is able to bind IFN-I with just slightly lower affinity than the complete protein, and it is able to block IFN-I effects in vitro. Although the interaction of these molecules with murine IFN-I have been studied more extensively in the present invention, the experiments conducted herein are a proof of concept assay revealing that D3 is able to block hIFNα action in vitro. Furthermore, previously calculated affinities of B18 for human and murine IFN-I are almost equivalent (Fernández de Marco et al., 2010 (cited above)). Thus, this relatively small molecule represents a promising therapeutic candidate for interferonopathies in humans.

An advantage of viral IFN-I decoy receptors over monoclonal antibodies is their ability to bind multiple forms of IFN-I with high affinity. A possible explanation for the lack of success in blocking IFN-I with monoclonal antibodies is that IFN-β and other IFN-α isoforms may remain active after treatment, a problem that vIFNα/βBPs solves. Moreover, D3 represents a small version of vIFNα/βBPs that could be humanized more easily.

Furthermore, by using the B18 protein own GAG-binding motives, both the minimal and the complete one (GAG-BD and D1), and the one from MC54L, another poxvirus protein, it was shown in the present invention that the addition of a cell surface binding domain to D3 increases its immunomodulatory activity and improves its therapeutic potential. Paradoxically, the lack of surface binding ability of the original D3 domain could also be beneficial in the treatment of IFN-I disorders, as it might allow the decoy receptors to exert their function at more distant tissues.

As opposed to Etanercept in the case of anti-TNFa therapies, there are currently no soluble receptors used in the clinic to block IFN-I. The present invention generates a collection of small soluble receptors with differential affinities for IFN-I and the cellular surface, resulting in the development of novel therapeutics for IFN-I mediated autoimmune diseases.

## Claims

1. An isolated amino acid sequence comprising an amino acid sequence having a sequence identity of at least 70, 80, 90, 91, 92, 93, 94, 95, 96, 97, 98, 99 o 100% with the sequence SEQ ID NO: 1, or the nucleotide sequence encoding thereof, for use as a medicament, preferably, for use in the treatment of an autoinflammatory disorder in a subject, with the proviso that the isolated amino acid sequence is not the sequence SEQ ID NO: 2, or SEQ ID NO: 3.

2. An amino acid sequence for use according to claim 1, further comprising a cell surface binding domain, preferably, the cell surface binding domain is a Glycosaminoglycan GAG-binding domain.

3. An amino acid sequence for use according to claim 1 or 2, wherein the cell surface binding domain is
(a) an amino acid sequence having a sequence identity of at least 70, 80, 90, 91, 92, 93, 94, 95, 96, 97, 98, 99 o 100% with the sequence SEQ ID NO: 4,
(b) an amino acid sequence having a sequence identity of at least 70, 80, 90, 91, 92, 93, 94, 95, 96, 97, 98, 99 o 100% with the sequence SEQ ID NO: 5, or
(c) an amino acid sequence having a sequence identity of at least 70, 80, 90, 91, 92, 93, 94, 95, 96, 97, 98, 99 o 100% with the sequence SEQ ID NO: 6.

4. An amino acid sequence for use according to any one of claims 1 to 3, wherein the isolated amino acid sequence comprises a linker between the sequence SEQ ID NO: 1 and the cell surface binding domain.

5. An amino acid sequence for use according to any one of claims 1 to 4, wherein the amino acid sequence comprises the sequence SEQ ID NO: 11, SEQ ID NO: 13 or SEQ ID NO: 15.

6. An amino acid sequence for use according to any one of claims 1 to 5, wherein the autoinflammatory disorder is an interferonopathy, preferably, the interferonopathy is selected from the group consisting of systemic lupus erythematosus, Aicardi-Goutieres syndrome, Candle (chronic atypical neutrophilic dermatosis with lipodystrophy and elevated temperature), Savi (STING-associated vasculopathy with onset in infancy), Spencd (spondyloenchondro-dysplasia with immune dysregulation) or Singleton-Merten syndrome.

7. An amino acid sequence for use according to any one of claims 1 to 6, wherein the nucleotide sequence comprises the sequence SEQ ID NO: 12. SEQ ID NO: 14 or SEQ ID NO: 16.

8. A Fusion protein comprising
(i) An amino acid sequence comprising an amino acid sequence having a sequence identity of at least 70, 80, 90, 91, 92, 93, 94, 95, 96, 97, 98, 99 o 100% with the sequence SEQ ID NO: 1, and
(ii) a cell surface binding domain,
with the proviso that the fusion protein is not the sequence SEQ ID NO: 17.

9. Fusion protein according to claim 8, wherein the cell surface binding domain is
(a) an amino acid sequence having a sequence identity of at least 70, 80, 90, 91, 92, 93, 94, 95, 96, 97, 98, 99 o 100% with the sequence SEQ ID NO: 4,
(b) an amino acid sequence having a sequence identity of at least 70, 80, 90, 91, 92, 93, 94, 95, 96, 97, 98, 99 o 100% with the sequence SEQ ID NO: 5, or
(c) an amino acid sequence having a sequence identity of at least 70, 80, 90, 91, 92, 93, 94, 95, 96, 97, 98, 99 o 100% with the sequence SEQ ID NO: 6.

10. Fusion protein according to claims 8 or 9, wherein the isolated amino acid sequence comprises a linker between the sequence SEQ ID NO: 1 and the cell surface binding domain.

11. Fusion protein according to any one of claims 8 to 10, wherein the amino acid sequence comprises the sequence SEQ ID NO: 11, SEQ ID NO: 13 or SEQ ID NO: 15.

12. An isolated polynucleotide comprising a nucleotide sequence encoding a fusion protein according to any one of claims 8 to 12, with the proviso that the nucleotide sequence is not the sequence SEQ ID NO: 18.

13. A vector or a cell comprising an isolated polynucleotide according to claim 12.

14. A composition comprising a fusion protein according to any one of claims 8 to 11, an isolated polynucleotide according to claim 12, or a vector or cell according to claim 13.

15. A fusion protein according to any one of claims 8 to 11, an isolated polynucleotide according to claim 12, a vector or cell according to claim 13, or a composition according to claim 14, for use as a medicament, preferably, for use in the treatment of an autoinflammatory disorder, more preferably, for use in the treatment of an interferonopathy, even more preferably, the interferonopathy is selected from the group consisting of systemic lupus erythematosus, Aicardi-Goutieres syndrome, Candle (chronic atypical neutrophilic dermatosis with lipodystrophy and elevated temperature), Savi (STING-associated vasculopathy with onset in infancy), Spencd (spondyloenchondro-dysplasia with immune dysregulation) or Singleton-Merten syndrome.
